# EUROPEAN PATENT APPLICATION

(11) **EP 3 766 477 A1**
(43) Date of publication of application: **20.01.2021**
(21) Application number: 19767915.2
(22) Date of filing: 15.03.2019
(51) Int. Cl.: A61K 8/44, A61K 8/02, A61K 8/31, A61K 8/34, A61K 8/36, A61K 8/37, A61K 8/86, A61K 8/891, A61Q 1/14, A61Q 19/00, A61Q 19/02, A61Q 19/08, A61Q 19/10

(54) **SOLID-FORM COSMETIC**

(30) Priority: 16.03.2018 JP 2018049415
(71) Applicant: Kokyu Alcohol Kogyo Co., Ltd., Narita-shi Chiba 287-0225 (JP)
(72) Inventor: OMURA, Takayuki, Narita-shi Chiba 287-0225 (JP); ISSHIKI, Mitsu, Narita-shi, Chiba 287-0225 (JP); ARAHIRA, Nana, Narita-shi, Chiba 287-0225 (JP); LORENZI, Christel, 69009 Lyon (FR)
(74) Representative: Cabinet Nuss
(86) International application number: PCT/JP2019/010775
(87) International publication number: WO 2019/177140

(57) **Abstract**

[Object]

The present invention relates to leave-off type or leave-on type solid form cosmetics, and provide solid form cosmetics that are excellent in cleansing or skincare performance and has high transmittance.

[Solution]

A solid form cosmetic comprising a gelling agent comprising dibutyl lauroyl glutamide and/or dibutyl ethyl hexanoyl glutamide, oil agent, surfactant and water. A solid form cosmetic, wherein the oil agent is a hydrocarbon oil, an ester oil having no hydroxy group, and/or a silicone oil which is not nonpolar..

## Description

### [Technical Field]

The present invention relates to solid form cosmetics.

### [Background Arts]

So far, solid form cosmetics using dibutyl lauroyl glutamide and dibutyl ethyl hexanoyl glutamide as gelling agents have been known.

Patent Document 1 discloses gelling agents comprising dibutyl lauroyl glutamide and dibutyl ethyl hexanoyl glutamide and describes that they are excellent in gel strength and transparency.

Patent Document 2 discloses cosmetics comprising dibutyl lauroyl glutamide and dibutyl ethyl hexanoyl glutamide as amino acid-based oil gelling agent and also comprising polyamide resin, and describes that the brittleness of the cosmetics using amino acid-based oil gelling agent alone can be improved by combined use with polyamide resin to provide cosmetics that are excellent in usability and storage stability.

Patent Document 3 discloses usability modifiers for cosmetic products comprising ester compounds of tricyclo[5.2.1.0^{2,6}]decane, and describes that they are excellent in usability.

Patent Document 4 discloses ultraviolet screening cosmetic compositions in transparent stick type comprising bisalkyl(C14-18) amide (ethylene diamine/hydrogenated dimer dilinoleate) copolymers as polyamide gelling agents, dibutyl lauroyl glutamide and dibutyl ethyl hexanoyl glutamide as amino acid gelling agents, as well as organic ultraviolet absorbing agents and oils.

Patent Document 5 discloses oily composition comprising N-acyl amino acid derivatives such as dibutyl lauroyl glutamide and dibutyl ethyl hexanoyl glutamide, polyamide resin as well as liquid oils, wherein the oily composition will be solidified to turn into a stick preparation for lips.

Patent Document 6 discloses oily solid form cosmetics comprising one or more oils selected from dibutyl lauroyl glutamide, diglyceryl triisostearate, diisostearyl malate, decaglyceryl decaisostearate and hydroxystearyl isooctanate, and fumed silica.

Patent Document 7 discloses oily solid form cosmetics in stick form having an transparent exterior part and colored interior part in contact with each other, comprising in the exterior part dibutyl lauroyl glutamide, dibutyl ethyl hexanoyl glutamide and polyglycerin fatty acid ester wherein the polymerization degree of the glycerin is between 6 and 12 and the fatty acid is branched fatty acid having from 8 to 12 carbon atoms.

### [Prior Art References]

### [Patent References]

[Patent Document 1] JP4174994B
[Patent Document 2] WO 2009/139092
[Patent Document 3] JP 5663111B
[Patent Document 4] KR 1020140031503B
[Patent Document 5] WO 2010/128639
[Patent Document 6] JP 2015-124181A
[Patent Document 7] JP 2017-119698A

### [Summary of the Invention]

### [Problems to be solved by the Invention]

As described above, solid form cosmetics so far using amino acid gelling agents are all oily solid form cosmetics with limited uses. In such circumstance, the present inventors came up with an idea that a new type of solid form cosmetics which has high additional values and can be expected to have new uses such as cleansing cosmetics or skincare cosmetics may be provided in a solid form cosmetic using amino acid gelling agents, and based on this idea, the inventors began the development of such solid form cosmetics. Namely, the object of the present invention is to provide a new type of solid form cosmetic using an amino acid gelling agent for which new uses can be expected.

### [Means to Solve the Problems]

The present inventors have made an intensive investigation for the purpose of achieving said object and found that a solid form cosmetic having a new function suitable for cleansing cosmetics and skincare cosmetics can be obtained by combining the amino acid gelling agent with a surfactant, oil agent and water, and further proceeded the research and completed the present invention.

Namely, the present invention relates to followings:
[1] A solid form cosmetic comprising a gelling agent comprising dibutyl lauroyl glutamide and/or dibutyl ethyl hexanoyl glutamide, oil agent, surfactant and water.
[2] The solid form cosmetic according to [1], wherein the transmittance is 80 % or higher.
[3] The solid form cosmetic according to [1] or [2], wherein the oil agent is a hydrocarbon oil, an ester oil having no hydroxy group, and/or a silicone oil which is not nonpolar.
[4] The solid form cosmetic according to any one of [1] to [3], wherein HLB of the surfactant is between 8 and 11.
[5] The solid form cosmetic according to any one of [1] to [4], wherein the content of dibutyl lauroyl glutamide is between 0.5 and 10.0 wt%, and the content of dibutyl ethyl hexanoyl glutamide is between 0.5 and 10.0 wt%.
[6] The solid form cosmetic according to any one of [1] to [5], wherein the content of the surfactant is between 5.0 and 15.0 wt%.
[7] The solid form cosmetic according to any one of [1] to [6], wherein the content of water is between 0.01 and 10.0 wt%.
[8] The solid form cosmetic according to any one of [1] to [7], further comprising isostearic acid or octyldodecanol.
[9] The solid form cosmetic according to any one of [1] to [8], wherein the blending ratio of dibutyl lauroyl glutamide to dibutyl ethyl hexanoyl glutamide is between 75:25 and 25:75.
[10] The solid form cosmetic according to any one of [1] to [9], wherein DSC solidifying temperature of the oil is is 50 °C or higher in a mixture of 90.0 wt% of oil agent with 10.0 wt% of a mixture of 27 wt% of dibutyl lauroyl glutamide, 9 wt% of dibutyl ethyl hexanoyl glutamide and 64 wt% of isostearic acid.
[11] The solid form cosmetic according to any one of [1] to [10], further comprising an ester having a hydroxy group 10.0 wt% or less.
[12] The solid form cosmetic according to any one of [1] to [11], wherein the solid form cosmetic is a leave-off type cosmetic.
[13] The solid form cosmetic according to any one of [1] to [12], wherein the solid form cosmetic is a leave-on type cosmetic.

### [Effects of the Invention]

By combining a gelling agent comprising dibutyl lauroyl glutamide (GP-1, from AJINOMOTO CO.,INC.) and/or dibutyl ethyl hexanoyl glutamide (EB-21, from AJINOMOTO CO.,INC.), oil agent, surfactant and water, a solid form cosmetic having functions with high additional values such as moisturizing, cleansing and/or skincare effects which are not offered by solid form cosmetics using conventional amino acid-based gelling agents can be provided while maintaining functions of the solid form cosmetics using amino acid-based gelling agent such as aesthetic appearance and skin fitness.

Also, a solid form cosmetic with high transmittance can be provided by these combinations. In particular, a solid form cosmetic with high transmittance can be provided by selecting the oil agent (main oil agent) from hydrocarbon oil, ester oil having no hydroxy group, and/or silicone oil which is not nonpolar. Furthermore, a solid form cosmetic with high transmittance can be provided by using a surfactant with HLB in the range from 8 to 11.

The solid form cosmetics of the present invention may further comprising an ester having a hydroxy group from the perspective of facilitating introduction of water or aqueous ingredients. Introduction of water is effective in assisting introduction of aqueous ingredients, i.e., various drugs, e.g., whitening drugs.

Furthermore, as solid form cosmetics according to these combinations, a leave-off type solid form cosmetic having an excellent cleansing effect or excellent cleansing and skincare effects, or a leave-on type solid form cosmetic having an excellent skincare effect can be provided.

In general, gelling agents such as dibutyl lauroyl glutamide or dibutyl ethyl hexanoyl glutamide are used in oily cosmetics. According to the present invention, they were combined with water, which in turn allowed a successful incorporation of a surfactant and thereby successfully provided a leave-off type cleansing cosmetic with high cleansing effect or high cleansing effect and high skincare effects such as moisture retention. A leave-on type skincare cosmetic with high skincare effects was also successfully provided.

### [Brief Description of Drawings]

[Fig. 1] Figure 1 is a diagram showing a comparison of transmittance in the case of using various oils with different DSC solidifying temperatures.
[Fig. 2] Figure 2 is a diagram showing a comparison of transmittance in the case of using various surfactants with different HLB values.
[Fig. 3] Figure 3 is a diagram showing a comparison of the differences in cleansing performance by the presence or absence of water-like ingredients in cleansing cosmetics.
[Fig. 4] Figure 4 is a diagram showing a comparison of the differences in cleansing performance between the oil agents in cleansing cosmetics.
[Fig. 5] Figure 5 is a diagram showing the difference in cleansing performance by the difference of the blended amounts of the surfactant and various oil agents in cleansing cosmetics.
[Fig. 6] Figure 6 is a diagram showing the solidity, transmittance and cleansing performance in the case of using AJK-OD2046 and AJK-IS3613 as gelling agents.
[Fig. 7] Figure 7 is a diagram illustrating the method for obtaining moisturization data of a leave-on type cosmetic.
[Fig. 8] Figure 8 is a diagram showing the results of water measurement for different water-like ingredients.
[Fig. 9] Figure 9 is a diagram illustrating the method for obtaining moisturization data of a leave-on type cosmetic.
[Fig. 10] Figure 10 is a diagram showing the results of water measurement for different water-like ingredients.

### [Modes for Practicing the Invention]

Hereinbelow, the present invention will be explained in detail based on suitable embodiments of the present invention.

The "solid form" in solid form cosmetics of the present invention refers to, without limitation, a solid form that has solidity of certain degree such that it is capable of forming a stick and being applied to skin. In specific, it has solidity of preferably between 0.05 and 0.3, more preferably between 0.1 and 0.2.

Besides, in measurement of solidity in the present invention, samples were placed in a thermostatic chamber at 25 °C for 24 hours, and measurement was carried out under room temperature of 25 °C on EZ TEST EZ-SX of Shimadzu Corporation using a needle-shape tool with 1 mm diameter and 30 mm length, at 10 mm/min needle-penetrating rate and 10mm strokes. The maximum value of solidity was considered as the solidity value for that sample.

The content of dibutyl lauroyl glutamide is preferably between 0.5 and 10.0 wt%, more preferably between 1.0 and 8.0 wt%, most preferably between 2.0 and 7.0 wt% to the entire cosmetic.

The content of dibutyl ethyl hexanoyl glutamide is preferably between 0.5 and 10.0 wt%, more preferably between 0.5 and 7.0 wt%, most preferably between 0.5 and 5.0 wt%.

When dibutyl lauroyl glutamide (GP-1) and dibutyl ethyl hexanoyl glutamide (EB-21) are contained, their blending ratio is to be determined from the perspective of transmittance, while the blending ratio (GP-1:EB-21) is preferably between 75:25 and 25:75, more preferably between 75:25 and 50:50. By using these gelling agents in a blending ratio in this range, a cosmetic with high transparency can be produced.

In the present invention, moreover, by using a solubilizing agent, the preparation of the gelling agent can be performed in a gentle condition, i.e., 100°C or lower. As solubilizing agents, without limitation, a higher fatty acid such as isostearic acid, octyldodecanol, and cetanol can be used, preferably a higher fatty acid such as isostearic acid or octyldodecanol, particularly preferably isostearic acid or octyldodecanol can be used. From the perspective of transparency, it is particularly preferred to use isostearic acid or octyldodecanol.

The content of the solubilizing agent is not limited, but preferably between 3.0 and 30.0 wt%, more preferably between 5.0 and 20.0 wt%, most preferably between 5.0 and 17.0 wt% to the entire cosmetic.

As oil agents (main oil agents) in the present invention, without limitation, a hydrocarbon oil, an ester oil having no hydroxy group, and/or a silicone oil which is not nonpolar can be used. These oil agents preferably have solidifying temperature of 50°C or higher when it was made to be a mixture with 10.0 wt% of a mixture of dibutyl lauroyl glutamide (27.0 %) and dibutyl ethyl hexanoyl glutamide (9.0 %) and isostearic acid (64.0 %) (AJK-IS3613, from KOKYU ALCOHOL KOGYO Co., Ltd.). Besides, solidifying temperature in the present invention was measured using a differential scanning calorimeter DSC7000 (Hitachi High-Tech Science Corporation). Hereinbelow, solidifying temperature measured according to the condition described above will be referred to as "DSC solidifying temperature of the oil agent".

Specific examples of oil agents are shown below (the temperatures in parentheses are DSC solidifying temperature of the oil agent).

As hydrocarbon oils, without limitation, mineral oil (trade name: HICALL® K-230, from KANEDA Co., Ltd., 83°C), hydrogenated polyisobutene (trade name: PARLEAM®6, from NOF CORPORATION, 81°C), hydrogenated polyisobutene (trade name: PARLEAM®18, from NOF CORPORATION, 93°C), hydrogenated polyisobutene (trade name: PARLEAM®24, from NOF CORPORATION, 98°C), hydrogenated polyisobutene (trade name: PARLEAM®46, from NOF CORPORATION, 104°C), etc. can be used.

As ester oils having no hydroxy group, without limitation, isodecyl neopentanoate (trade name: NEOLIGHT 100P, from KOKYU ALCOHOL KOGYO Co., Ltd., 60°C), ethylhexyl isononanoate (trade name: ES108109, from KOKYU ALCOHOL KOGYO Co., Ltd., 66°C), isopropyl myristate (trade name: IPM-R, from KOKYU ALCOHOL KOGYO Co., Ltd., 66°C), hexyl laurate (trade name: KAK HL, from KOKYU ALCOHOL KOGYO Co., Ltd., 73°C), isononyl isononanate (trade name: KAK 99, from KOKYU ALCOHOL KOGYO Co., Ltd., 62°C), ethyl isostearate (trade name: EIS-V, from KOKYU ALCOHOL KOGYO Co., Ltd., 64°C), isotridecyl isononanate (trade name: KAK 139, from KOKYU ALCOHOL KOGYO Co., Ltd., 73°C), neopentyl glycol diethylhexanoate (trade name: KAK NDO, from KOKYU ALCOHOL KOGYO Co., Ltd., 67°C), isobutyl isostearate (trade name: KAK IBIS, from KOKYU ALCOHOL KOGYO Co., Ltd., 70°C), ethyl ethylhexanoate (trade name: CEH, from KOKYU ALCOHOL KOGYO Co., Ltd., 84°C), isostearyl neopentanoate (trade name: isostearyl neopentanoate, from KOKYU ALCOHOL KOGYO Co., Ltd., 74°C), neopentyl glycol dicaprate (trade name: NPDC, from KOKYU ALCOHOL KOGYO Co., Ltd., 67°C), bis-ethoxydiglycol succinate (trade name: HAIAQUEOUSTER DCS, from KOKYU ALCOHOL KOGYO Co., Ltd., 67°C), isocetyl myristate (trade name: ICM-R, from KOKYU ALCOHOL KOGYO Co., Ltd., 83°C), neopentylglycol diisononanoate (trade name: NPDIN, from KOKYU ALCOHOL KOGYO Co., Ltd., 65°C), tri(capryate/caprate) glyceryl (trade name: TCG-M, from KOKYU ALCOHOL KOGYO Co., Ltd., 67°C), octyldodecyl myristate (trade name: ODM, from KOKYU ALCOHOL KOGYO Co., Ltd., 86°C), triethylhexanoin (trade name: TOG, from KOKYU ALCOHOL KOGYO Co., Ltd., 70°C), hexyldecyl isostearate (trade name: ICIS, from KOKYU ALCOHOL KOGYO Co., Ltd., 86°C), isosteryl isostearate (trade name: ISIS, from KOKYU ALCOHOL KOGYO Co., Ltd., 87°C), tricyclodecanemethyl isononanoate (trade name: KAK TCIN, from KOKYU ALCOHOL KOGYO Co., Ltd., 58°C), trimethylolpropane triethylhexanoate (trade name: KAK TTO, from KOKYU ALCOHOL KOGYO Co., Ltd., 70°C), octyldodecyl stearoyloxystearate (trade name: RISOCAST ODSHS, from KOKYU ALCOHOL KOGYO Co., Ltd., 88 °C), diisopropyl dimer dilinoleate (trade name: KAK DADIP-R, 80°C), triisostearin (trade name: TISG, from KOKYU ALCOHOL KOGYO Co., Ltd., 77°C), trimethylolpropane triisostearate, from KOKYU ALCOHOL KOGYO Co., Ltd., 85 °C), pentaerythrityl tetraisostearate (trade name: KAK PTI, from KOKYU ALCOHOL KOGYO Co., Ltd., 87 °C), dipentaerythrityl hexaisononanoate (trade name: HAILUCENT DPIN6, from KOKYU ALCOHOL KOGYO Co., Ltd., 76°C), etc. can be used.

As silicone oils which are not nonpolar, without limitation, diphenyl siloxyphenyltrimethicone (trade name: KF-56A, from Shin-Etsu Chemical Co., Ltd., 60°C), diphenyldimethicone (trade name: KF-54, from Shin-Etsu Chemical Co., Ltd., 78°C), triphenyl dimethyldisiloxane (trade name: Silshine VP Fluid, from Momentive Performance Materials Japan, LLC, 83°C), polysilicon-32 (trade name: Silshine MP Fluid, from Momentive Performance Materials Japan, LLC, 88°C), etc. can be used.

From the perspective of viscosity or usability of the cosmetic, oil agent (main oil agent) is preferably one whose
molecular weight is 1000 or lower, particularly preferably 500 or lower.

In the present invention, the content of oil agent (main oil agent) is not limited, but preferably between 20 and 60 wt%, furthermore, preferably between 30 and 50 wt%.

Surfactants in the present invention is not limited, but, from the perspective of transparency, it is preferred to have HLB between 8 to 11, and in specific, Ceteth-5 (HLB8), Ceteth-7 (HLB10), Ceteth-10 (HLB11), Steareth-5 (HLB5), Steareth-6 (HLB8), Steareth-8 (HLB9), Steareth-10 (HLB11), Steareth-11 (HLB11), Steareth-12 (HLB11), Laureth-3 (HLB8), Laureth-5 (HLB10), Laureth-7 (HLB11), PPG-2 Deceth-5 (HLB9), PPG-2 Deceth-7 (HLB10), Beheneth-10 (HLB9), PEG-10 phytosterol (HLB9), Choleth-10 (HLB10), Choleth-15 (HLB11), Isoceteth-5 (HLB8), Isoceteth-10 (HLB11), Isosteareth-5 (HLB8), Isosteareth-10 (HLB11), Octyldodeceth-10 (HLB10), Decyltetradeceth-10 (HLB9), Decyltetradeceth-15 (HLB11), Hydrogenated Dimer Dilinoleth-20 (HLB11), PEG-5 stearate (HLB8), PEG-10 stearate (HLB11), PEG-6 isostearate (HLB9), PEG-8 isostearate (HLB10), PEG-10 isostearate (HLB11), PEG-30 hydrogenated castor oil (HLB9), PEG-40 hydrogenated castor oil (HLB11), Laureth-10 stearate (HLB8), Laureth-15 stearate (HLB9), Laureth-10 isostearate (HLB8), Steareth-12 stearate (HLB8),

PEG-8 dilaurate (HLB8), PEG-12 dilaurate (HLB10), PEG-16 dilaurate (HLB11), PEG-8 dilaurate (HLB8), PEG-12 distearate (HLB8), PEG-12 diisostearate (HLB8), PEG-12 dioleate (HLB8), PEG-5 glyceryl isostearate (HLB8), PEG-6 isostearate (HLB8), PEG-8 glyceryl isostearate (HLB10), PEG-10 glyceryl isostearate (HLB10), PEG-20 glyceryl triisostearate (HLB8), PEG-30 glyceryl triisostearate (HLB10), PEG-40 glyceryl isostearate (HLB11), PEG-10BG isostearate (HLB10), PEG-15BG diisostearate (HLB8), PEG-20 hydrogenated castor oil laurate (HLB8), PEG-30 hydrogenated castor oil laurate (HLB10), PEG-40 hydrogenated castor oil laurate (HLB11), PEG-20 hydrogenated castor oil isostearate (HLB8), PEG-30 hydrogenated castor oil isostearate (HLB9), PEG-40 hydrogenated castor oil isostearate (HLB11), PEG-40 hydrogenated castor oil triisostearate (HLB8), PEG50 hydrogenated castor oil triisostearate (HLB9), PEG-60 hydrogenated castor oil triisostearate (HLB10), PEG-20 trimethylolpropane trimyristate (HLB9), PEG-30 trimethylolpropane trimyristate (HLB11), PEG-20 trimethylolpropane triisostearate (HLB8), PEG-30 trimethylolpropane triisostearate (HLB10), PEG-40 trimethylolpropane triisostearate (HLB11), fatty acid polyglyceryl-10 tricocoate (HLB9), polyglyceryl-6 distearate (HLB8), polyglyceryl-10 tristearate (HLB8), polyglyceryl-6 diisostearate (HLB8), polyglyceryl-6 diisostearate (HLB8), polyglyceryl-10 diisostearate (HLB10), PCA (pyroglutamic acid) PEG-40 hydrogenated castor oil isostearate (HLB11), etc. can be used.

In the present invention, the content of the surfactant is not limited, but preferably between 5 and 20 wt%, more preferably between 8 and 15 wt%.

The blended amount of water used in the present invention is preferably between 0.01 and 10.0 wt%. Below 0.01 wt%, freshness, which is the effect of the present invention, cannot be obtained. On the other hand, over 10.0 wt% of water blending causes problems including becoming difficult to molding the stable shaping into a stick form, causing problems in solidity, or being ease to break at the time of application. By including water, the cleansing property and skincare property (moisturizing effect) are improved as compared to the conventional non-water-like, oily transparent solid form cosmetics.

As aqueous ingredients in the present invention, without limitation, plant extracts, plant essences, whitening drugs, amino acids or derivatives thereof, etc. can be used.

As plant extracts and essences, without limitation, *Angelica keiskei* essence, *Vigna angularis* leaf essence, *Uncaria gambir* essence, *Althaea* sp.root essence, *Arnica* sp.flower essence, aloe essence, aloe vera essence, *Bidens pilosa var. minor* essence, ginkgo leaf essence, nettle leaf essence, fennel fruit essence, *Rosa multiflora* fruit essence, *Rabdosia japonica* leaf/stalk essence, *Scutellaria baicalensis* leaf essence, Amur cork tree bark essence, *Hypericum erectum* flower/leaf/stalk essence, *Nasturtium officinale* leaf/stalk essence, *Laminaria japonica* essence, *Artemisia indica var. maximowiczii* leaf essence, *Fucus sp.* essence, chamomile flower essence, wild oat grain essence, *Artemisia capillaris* flower essence, gardenia fruit essence, *Sasa veitchii* leaf essence, *Sophora flavescens* root essence, black tea essence, burdock root essence, rice bran root essence, pomegranate flower essence, *Opuntia streptacantha* essence, *Saponaria officinalis* leaf essence, sage leaf essence, *Rehmannia glutinosa* root essence, perilla leaf essence, *Paeonia lactiflora* root essence, *Houttuynia cordata* essence, ginger rhizome essence, *Acorus calamus* root essence, melon placenta essence, *Betula pubescens* bark essence, *Lithospermum erythrorhizon* root essence, *Equisetum arvense* essence, *Hedera helix* leaf/stalk essence, *Achillea millefolium* essence, peppermint leaf essence, *Tilia cordata Mill.* flower essence, *Malva mauritiana L.* flower essence, *Swertia japonica* essence, *Morus Alba* root bark essence, soybean seed essence, wild thyme essence, *Camellia sinensis* leaf essence, *Camellia sinensis* flower essence, clove essence, *Citrus unshiu* hull essence, red pepper essence, *Angelica acutiloba* root essence, *Calendula officinalis* flower essence, bitter orange hull essence, Panax ginseng root essence, *Rosa canina* fruit essence, parsley essence, hamamelis essence, *Rosa centifolia* flower essence, *Eriobotrya japonica* leaf essence, *Pleurotus cornucopiae var. citrinopileatus* essence, *Ruscus aculeatus* root essence, grape leaf essence, sponge gourd essence, safflower flower essence, *Typha domingensis* seed essence, *Paeonia suffruticosa* essence, *Humulus lupulus* essence, horse chestnut essence, rosemary leaf essence, melissa leaf essence, melilot essence, peach leaf essence, *Centaurea cyanus* flower essence, *Saxifraga stolonifera* essence, eucalyptus leaf essence, *Lilium candidum L.* root essence, adlay seed essence, lavender flower essence, grapevine essence, red clover flower essence, *Syzygium jambos* leaf essence, rosemary leaf essence, roman chamomile flower essence, burnet essence, *Adiantum capillus-veneris L.* leaf essence, *Tephrosia purpurea* leaf essence, *Leucas cephalotes* leaf essence, *Psoralea corylifolia* fruit essence, *Bergenia ligulata* root essence, etc. can be used.

As whitening drugs, drugs which are generally used in any cosmetic products can be blended in a range such that there will be no influence on stability. In specific, without limitation, arbutin, ellagic acid, Chamomilla ET, t-AMCHA, tranexamic acid, vitamin C derivatives such as ascorbic acid glucoside, magnesium ascorbyl phosphate, disodium ascorbyl sulfate and 3-O-cetyl ascorbic acid, placenta essence, linoleic acid, Rucinol®, potassium 4-methoxysalicylate, etc. can be used.

As amino acids, arginine, glycine, glutamic acid, sarcosine, valine, threonine, serine, leucine, proline, histidine, alanine, etc. can be used, and as amino acid derivatives, the dipeptides and polypeptides, etc. derived from these amino acids can be used.

Furthermore, in the present invention, oil-soluble drugs can be blended, e.g., without limitation, ceramides and ceramide derivatives such as ceramide NG, ceramide NP and phytosphingosine, vitamin A and vitamin A derivatives such as retinol, retinol acetate, retinol linoleate and hydrogenated retinol, vitamin B and vitamin B derivatives such as pyridoxine dicapryate, pyridoxine dipalmitat, 2, 4-dicarboethoxy ethyl pantothenate and pyridoxine tris-hexyldecanoate, and vitamin C and vitamin C derivatives such as ascorbyl dipalmitate, ascorbyl tetrahexyl decanoate and tocopheryl retinoate can be used.

Moreover, oil-soluble plant essences can be blended, e.g., without limitation, oil-soluble Arnica essence (arnica flower essence/mineral oil mixture), oil-soluble chamomilla essence (chamomile flower essence/sunflower seed oil mixture, chamomile flower essence/tri(capryate/caprate) glyceryl mixture, chamomile flower essence/mineral oil mixture), oil-soluble lithospermum root essence (Lithospermum Radix essence/squalane mixture), oil-soluble Equisetum arvense essence (Equisetum arvense essence/sunflower seed oil mixture), oil-soluble linden essence (Tilia europaea flower/leaf essence/sunflower seed oil mixture), oil-soluble Achillea millefolium essence (Achillea millefolium essence/sunflower seed oil mixture), oil-soluble sage essence (sage leaf essence/sunflower seed oil mixture), oil-soluble ginseng essence (Panax ginseng leaf essence/squalane mixture), oil-soluble horse chestnut essence (horse chestnut essence/sunflower seed oil mixture), oil-soluble peach leaf essence (peach leaf essence/squalane mixture), oil-soluble rosemary essence (rosemary leaf essence/sunflower seed oil mixture, rosemary leaf essence/mineral oil/apricot kernel oil mixture), etc. can be used.

In the present invention, for the purpose of facilitating introduction of water and improving cleansing or skincare effect, an ester having a hydroxy group can be blended in the composition 10% or less. It is not preferred that the content is over 10 %, because it causes a problem in transparency, which is a characteristic of the present invention.

As esters having a hydroxy group in the present invention, without limitation, sodium isostearoyl lactate, diisostearyl malate, hydroxystearyl ethylhexyl, octyldodecyl stearoyloxy stearate, etc. can be used.

The solid form cosmetics of the present invention can be used as either a leave-on type or leave-off type cosmetic.

A leave-off type cosmetic is a cosmetic that needs to be wiped off or washed off after being applied, and, without limitation, is a cosmetic used as a cleansing cosmetic. On the other hand, a leave-on type cosmetic is a cosmetic that does not need to be wiped off after being applied, and, without limitation, is a cosmetic used for anti-aging, whitening or moisturing. In the case of leave-on type cosmetics, the content of the surfactant is preferably 15.0 wt% or less, more preferably between 5.0 and 15.0 wt%.

In the present specification, a "water-like ingredient" is an ingredient contained in a cosmetic, and includes, without limitation, water, BG (buthylene glycol), etc.

In the present specification, an "aqueous ingredient" is an water-soluble effective ingredient that has cleansing or skincare effect, and includes those having whitening or moisturizing effect, etc.

### Various Assessments

### 1. Assessments of Transmittance

### <Measuring Methods>

Transmittance was measured using JASCO spectrometer V-650 in absorbance wavelength range between 350 nm and 800 nm. The average value between 380 nm and 750 nm was considered as the transmittance.

### (1) Difference by the type of oil agent (main oil agent)

Transmittances using various oil agents in Table 2 according to the formulations in Table 1 were measured.

### [Table 1]

**Table 1. Formulation to measure the difference in transmittance by different oil agents**

| Product Name | Cosmetic Ingredient Label Name | wt% | Supplier |
|---|---|---|---|
| Purified water | Water | 5 | - |
| HAISUGARCANE BG | BG | 5 | KOKYU ALCOHOL KOGYO Co., Ltd. |
| RISOREX PGIS23 | Polyglyceryl-2 triisostearate | 10 | KOKYU ALCOHOL KOGYO Co., Ltd. |
| EMALEX GWIS-320EX | PEG-20 glyceryl-2 triisostearate | 25 | NIHON EMULSION Co., Ltd. |
| AJK-IS3613 | Isostearic acid, dibutyl lauroyl glutamide, dibutyl ethyl hexanoyl glutamide | 10 | KOKYU ALCOHOL KOGYO Co., Ltd. |
| Various oil agents | | 45 | - |

### [Table 2]

**Various oil agents in Table 1**

| Product Name | Cosmetic Ingredient Label Name | Supplier |
|---|---|---|
| HICALL®K230 | Mineral oil | KANEDA Co., Ltd. |
| PARLEAM®6 | Hydrogenated polyisobutene | NOF CORPORATION |
| KAK 99 | Isononyl isononanoate | KOKYU ALCOHOL KOGYO Co., Ltd. |
| TCG-M | Tri(caprylate/caprate) glyceryl | KOKYU ALCOHOL KOGYO Co., Ltd. |
| KAK TCIN | Tricyclodecane methyl isononanoate | KOKYU ALCOHOL KOGYO Co., Ltd. |
| Rice bran oil | Rice bran oil | Boso Oil and Fat, Co., Ltd. |

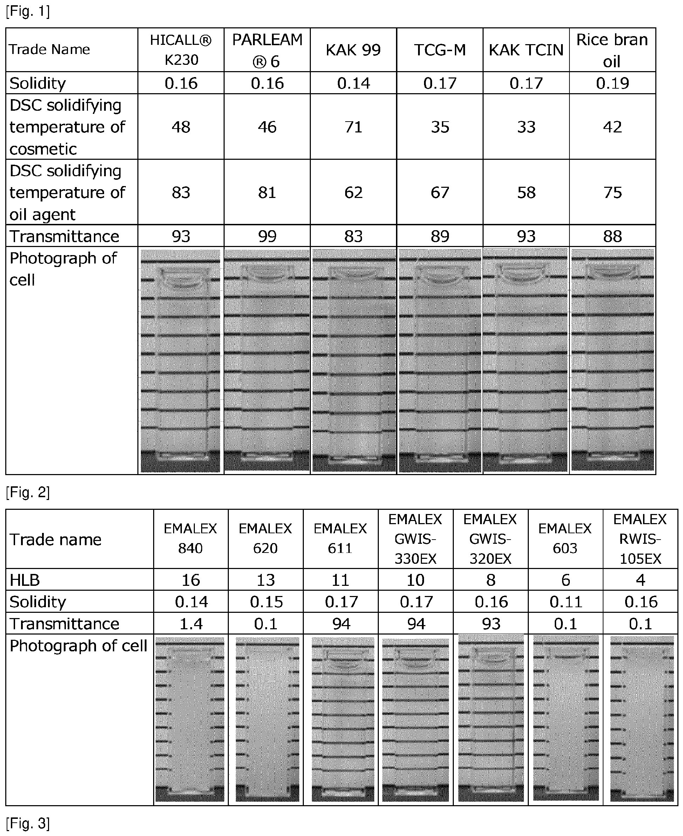

The results for the oil agents whose transmittances were 80% or more are shown in Fig. 1.

### <Assessment Results>

High transmittance was observed when using an oil agent having DSC solidifying temperature (DSC solidifying temperature of the oil agent) of 50°C or higher in a mixture of 10 wt% of AJK-IS with 90 wt% of various oil agents.

### (2) Difference by HLB values of surfactants

Transmittances using various surfactants in Table 4 according to the formulations in Table 3 were measured.

### [Table 3]

**Table 3. Formulation to measure the difference in transmittance by different surfactants**

| Product Name | Cosmetic Ingredient Label Name | wt% | Supplier |
|---|---|---|---|
| Purified water | Water | 5 | - |
| HAISUGARCANE BG | BG | 5 | KOKYU ALCOHOL KOGYO Co., Ltd. |
| RISOREX PGIS23 | Polyglyceryl-2 triisostearate | 10 | KOKYU ALCOHOL KOGYO Co., Ltd. |
| AJK-IS3613 | isostearic acid, dibutyl lauroyl glutamide, dibutyl ethyl hexanoyl glutamide | 10 | KOKYU ALCOHOL KOGYO Co., Ltd. |
| HICALL®K230 | Mineral oil | 45 | KANEDA Co., Ltd. |
| Various surfactants | - | 25 | - |

### [Table 4]

**Table 4. Various surfactants in Table 3**

| Product Name | Cosmetic Ingredient Label Name | HLB | Supplier |
|---|---|---|---|
| EMALEX 840 | PEG-40 stearate | 16 | NIHON EMULSION Co., Ltd. |
| EMALEX 620 | Steareth-20 | 13 | |
| EMALEX 611 | Steareth-11 | 11 | |
| EMALEX GWIS-330EX | PEG-30 glyceryl tristearate | 10 | |
| EMALEX GWIS-320EX | PEG-20 glyceryl tristearate | 8 | |
| EMALEX 603 | Steareth-3 | 6 | |
| EMALEX RWIS-105EX | PEG-5 isostearate hydrogenated castor oil | 4 | |

Results are shown in Fig. 2. High transmittance was observed when HLB value of the surfactant was in a range between 8 and 11.

### 2. Assessment of cleansing performance

### <Measuring Methods>

(i) Using KES-SE (KATO TECH, Co., Ltd.), a tester for the feeling of friction, a lipstick and sample were reciprocated on an artificial leather in this order.
Conditions:

| | |
|---|---|
| Measurement temperature: | sample platform, 22.0°C |
| Chamber condition: | temperature 23°C, humidity 50% |
| Number of application: | Lipstick, 1 reciprocation |
| | Sample, 5 reciprocations |
| Loading: | 25 g |
| Rate: | 10 mm/sec |

(ii) The applied surface was wiped off with 3 strokes of finger-held Kimwipe®
(iii) The area 1 cm inside from the outer ends of the cleansed site (230 mm²) was photographed with digital microscope (KEYENCE CORPORATION).
(iv) Percentage of removed lipstick was calculated from the residual amount of the lipstick on the artificial leather by image analysis software.

### <Assessment Results>

### (1) Difference by the presence/absence of water

Cleansing cosmetics of the following formulations were prepared and their cleansing performances were assessed.

### [Table 5]

**Table 5. Formulation for assessing differences in cleansing performance by the presence/absence of water-like ingredient**

| Product Name | Cosmetic Ingredient Label Name | Supplier | wt. % | |
|---|---|---|---|---|
| | | | With water-like ingredient | No water-like ingredient |
| Purified water | Water | - | 0 | 4 |
| HAISUGARCANE BG | BG | KOKYU ALCOHOL KOGYO Co., Ltd. | 0 | 1 |
| EMALEX GWIS-330EX | PEG-30 glyceryl-2 triisostearate | NIHON EMULSION Co., Ltd. | 10 | 10 |
| AJK-IS3613 | Isostearic acid, dibutyl lauroyl glutamide, dibutyl ethyl hexanoyl glutamide | KOKYU ALCOHOL KOGYO Co., Ltd. | 10 | 10 |
| HICALL®K230 | Mineral oil | KANEDA Co., Ltd. | 52 | 47 |
| IPM-R | Isopropyl myristate | KOKYU ALCOHOL KOGYO Co., Ltd. | 28 | 28 |

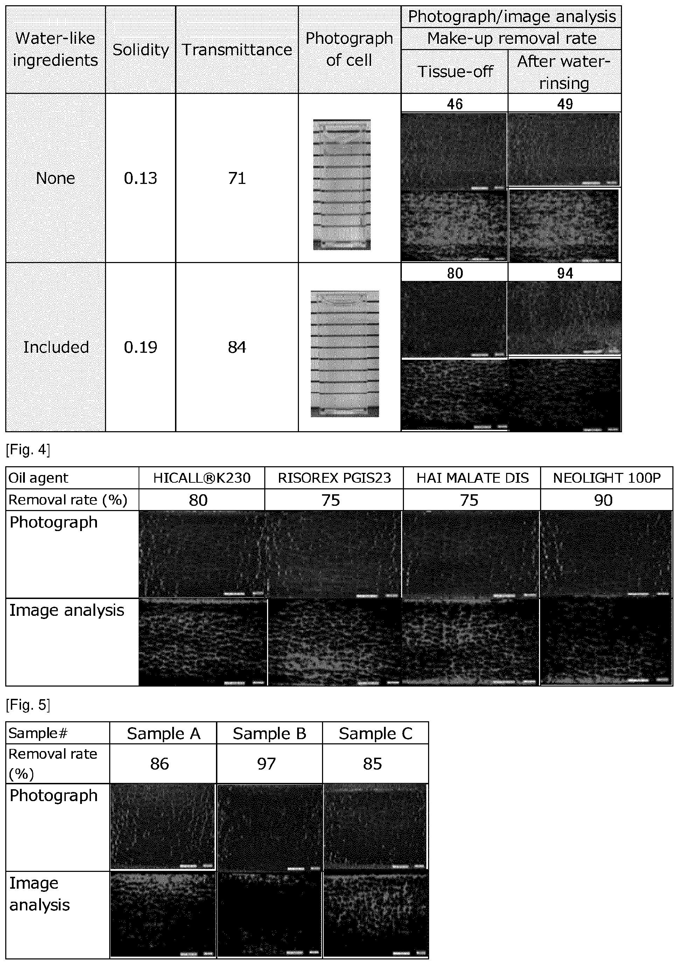

Results are shown in Fig. 3.

From the results in Fig. 3, cleansing cosmetics comprising water-like ingredients had better cleansing performances and higher transmittances than cosmetics comprising no water-like ingredient in both tissue-off and wash-off cases.

### (2) Difference by the type of oil agent

As shown in Table 6, cleansing cosmetics of formulation comprising mineral oil as the main oil agent (45 wt%) with an addition of 10 wt% of each oil agent in Table 7 were prepared to assess their cleansing performances.

### [Table 6]

**Table 6. Formulation for assessing cleansing performance by different types of oils**

| Product Name | Cosmetic Ingredient Label Name | wt. % | Supplier |
|---|---|---|---|
| Purified water | Water | 5 | - |
| HAISUGARCANE BG | BG | 5 | KOKYU ALCOHOL KOGYO Co., Ltd. |
| EMALEX GWIS-320EX | PEG-20 glyceryl-2 triisostearate | 25 | NIHON EMULSION Co., Ltd. |
| AJK-IS3613 | Isostearic acid, dibutyl lauroyl glutamide, dibutyl ethyl hexanoyl glutamide | 10 | KOKYU ALCOHOL KOGYO Co., Ltd. |
| HICALL®K230 | Mineral oil | 45 | KANEDA Co., Ltd. |
| Various oil agents | - | 10 | - |

### [Table 7]

**Table 7.Various oils in Table 6**

| Product Name | Cosmetic Ingredient Label Name | Supplier |
|---|---|---|
| RISOREX PGIS23 | Polyglyceryl-2 triisostearate | KOKYU ALCOHOL KOGYO Co., Ltd. |
| HAI MALATE DIS | diisostearyl malate | KOKYU ALCOHOL KOGYO Co., Ltd. |
| NEOLIGHT 100P | isodecyl neopentanoate | KOKYU ALCOHOL KOGYO Co., Ltd. |
| H ICALL®K230 | Mineral oil | KANEDA Co., Ltd. |

Results are shown in Fig. 4.

It was found that cleansing performance is slightly better using NEOLIGHT 100P.

### (3) Difference by the blended amount of the surfactant

Cleansing cosmetics were prepared according to the formulations in Table 8 and their cleansing performances were assessed.

### [Table 8]

**Table 8. Formulation for assessing cleansing performance by different amounts of surfactant and oil agents blended**

| Product Name | Cosmetic Ingredient Label Name | Supplier | wt. % | | |
|---|---|---|---|---|---|
| | | | Sample A | Sample B | Sample C |
| Purified water | Water | - | 5 | 4 | 5 |
| HAISUGARCANE BG | BG | KOKYU ALCOHOL KOGYO Co., Ltd. | 5 | - | - |
| RISOREX PGIS23 | Polyglyceryl-2 triisostearate | KOKYU ALCOHOL KOGYO Co., Ltd. | 10 | - | 5 |
| EMALEX GWIS-320EX | PEG-20 glyceryl-2 triisostearate | NIHON EMULSION Co., Ltd. | **25** | **7** | **7** |
| RISOREX PGIS21 | Polyglyceryl-2 isostearate | KOKYU ALCOHOL KOGYO Co., Ltd. | - | 4 | - |
| AJK-IS3613 | Isostearic acid, dibutyl lauroyl glutamide, dibutyl ethyl hexanoyl glutamide | KOKYU ALCOHOL KOGYO Co., Ltd. | 10 | 10 | 10 |
| H ICALL®K230 | Mineral oil | KANEDA Co., Ltd. | - | 30 | - |
| NEOLIGHT 100P | Isodecyl neopentanoate | KOKYU ALCOHOL KOGYO Co., Ltd. | 30 | - | 30 |
| KAK TCIN | Tricyclodecane methyl isononanoate | KOKYU ALCOHOL KOGYO Co., Ltd. | 10 | | - |
| HAILUCENT DPIN6 | Dipentaerythrityl hexaisononanoate | KOKYU ALCOHOL KOGYO Co., Ltd. | 5 | - | - |
| TCG-M | Tri(caprylate/caprate) glyceryl | KOKYU ALCOHOL KOGYO Co., Ltd. | - | 15 | 8 |
| IPM-R | Isopropyl myristate | KOKYU ALCOHOL KOGYO Co., Ltd. | - | 30 | - |
| KAK PTI | Pentaerythrityl tetraisostearate | KOKYU ALCOHOL KOGYO Co., Ltd. | - | - | 30 |
| HAILUCENT ISDA | (Polyglyceryl-2 isostearate/dimer dilinoleate) copolymer | KOKYU ALCOHOL KOGYO Co., Ltd. | - | - | 5 |

Results are shown in Fig. 5.

From the results in Fig. 5, it was found that cleansing performance depends rather on the type of the oil agent than on the blended amount of the surfactant.

### (4) Difference by different solubilizing agent in the gelling agent

Using AJK-OD2046 and AJK-IS3613 as the gelling agent, cleansing cosmetics according to various formulations in Table 9 were prepared and their solidity, transmittance, and cleansing performances were assessed.

### [Table 9]

**Table 9. Formulation for assessing various physical properties for different solubilizing agents in gelling agents**

| Product Name | Cosmetic Ingredient Label Name | Supplier | wt% | |
|---|---|---|---|---|
| | | | AJK-OD2046* | AJK-IS3613 |
| Purified water | Water | - | 4 | 4 |
| HAISUGARCANE BG | BG | KOKYU ALCOHOL KOGYO Co., Ltd. | 1 | 1 |
| EMALEX GWIS-320EX | PEG-20 glyceryl triisostearate | NIHON EMULSION Co., Ltd. | 10 | 10 |
| Various gelling agents | - | KOKYU ALCOHOL KOGYO Co., Ltd. | 20 | 10 |
| HICALL®K230 | Mineral oil | KANEDA Co., Ltd. | 37 | 47 |
| EMALEX GWIS-330EX | PEG-30 glyceryl triisostearate | NIHON EMULSION Co., Ltd. | 28 | 28 |

| | | | | |
|---|---|---|---|---|
| AJK-OD2046*: Mixture of octyldodecanol, dibutyl lauroyl glutamide and dibutyl ethyl hexanoyl glutamide | | | | |

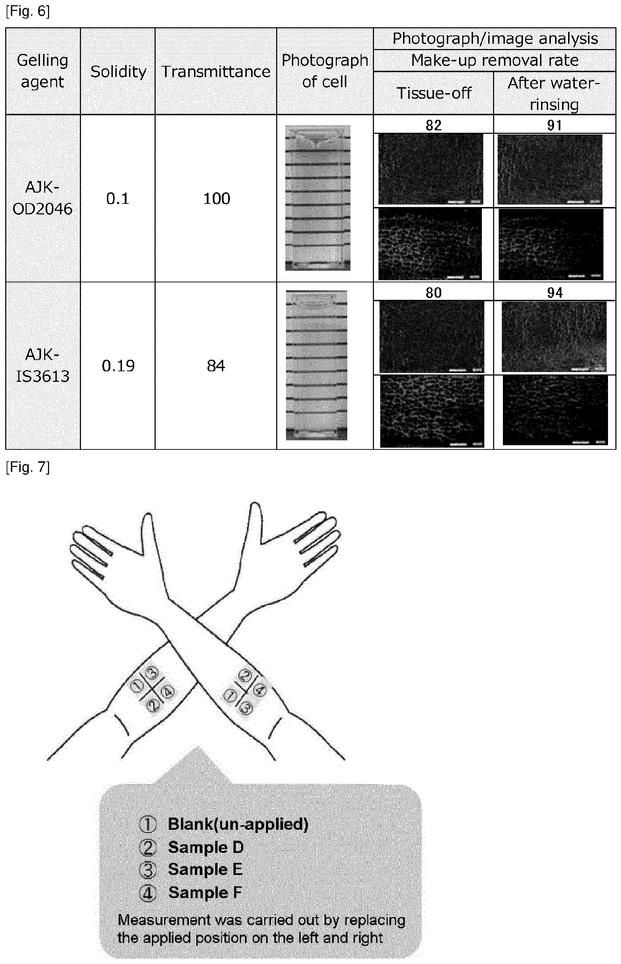

Results are shown in Fig. 6.

From the results in Fig. 6, in the case using the gelling agent of either AJK-OD2046 or AJK-IS3613, desired physical property values were shown in solidity, transmittance and cleansing performance.

### 3. Assessment of moisturizing performance

(1) Skincare cosmetics were prepared according to the formulations in Table 10 and their moisturizing performances were assessed.

### [Table 10]

**Table 10. Formulation for assessing the difference in moisturizing performance by the presence/absence of water-like ingredients**

| Product Name | Cosmetic Ingredient Label Name | Supplier | wt% | | |
|---|---|---|---|---|---|
| | | | Sample D | Sample E | Sample F |
| Purified water | Water | - | 0 | 2 | 2 |
| HAISUGARCANE BG | BG | KOKYU ALCOHOL KOGYO Co., Ltd. | 0 | 0 | 8 |
| triol VE | Glycerin | KOKYU ALCOHOL KOGYO Co., Ltd. | 0 | 5 | 0 |
| EMALEX GWIS-330EX | PEG-30 glyceryl triisostearate | NIHON EMULSION Co., Ltd. | 10 | 10 | 10 |
| AJK-IS3613 | Isostearic acid, dibutyl lauroyl glutamide, dibutyl ethyl hexanoyl glutamide | KOKYU ALCOHOL KOGYO Co., Ltd. | 10 | 10 | 10 |
| HICALL®K230 | Mineral oil | KANEDA Co., Ltd. | 52 | 45 | 27 |
| IPM-R | Isopropyl myristate | KOKYU ALCOHOL KOGYO Co., Ltd. | 28 | 28 | 43 |
| Solidity | | | 0.13 | 0.1 | 0.13 |

### <Measuring Methods>

(i) As shown in Fig. 7, each sample is applied to a forearm in an area of 2 cm x 2 cm so as to slid in one direction (3 slides).
(ii) Leave for 30 min.
(iii) The applied surface was wiped off in one direction with 3 strokes of finger-held Kimwipe® to remove excessive oil agent.
(iv) Water content is measured every 2 hours (no regulation in pretreatment or environment where the subject spends his/her time; ambient environment (temperature and humidity) at the time of measurement are recorded.) (no regulation in pretreatment or environment where the subject spends his/her time)

### <Assessment Results>

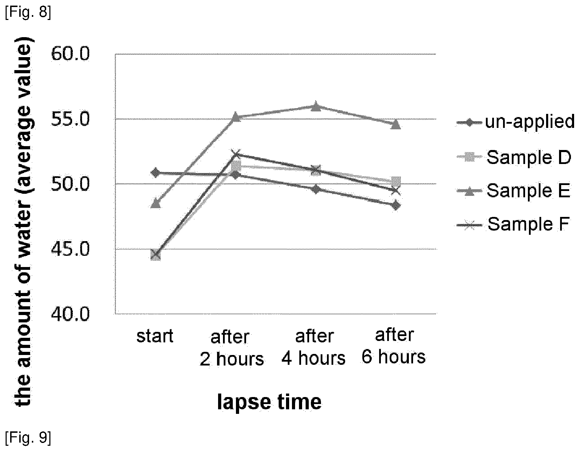

Results are shown in Fig. 8.

Samples were compared with un-applied control after equal time course, and Sample E (5% glycerin blended) showed a result with significant difference in moisturizing effect after 4 hours and after 6 hours.
(2) Skincare cosmetics were prepared according to the formulations in Table 11 and their moisturizing performances were assessed.

### [Table 11]

**Table 11. Formulation 2 for assessing the difference in moisturizing performance by the presence/absence of water-like ingredients**

| Product Name | Cosmetic Ingredient Label Name | Supplier | wt% | | |
|---|---|---|---|---|---|
| | | | Sample G | Sample H | Sample J |
| Purified water | Water | - | 0 | 1 | 2.5 |
| HAISUGARCANE BG | BG | KOKYU ALCOHOL KOGYO Co., Ltd. | 0 | 0 | 3 |
| triol VE | Glycerin | KOKYU ALCOHOL KOGYO Co., Ltd. | 0 | 5 | 0 |
| Amalty®MR-50 | Maltitol | Mitsubishi Shoji Foodtech Co., Ltd. | 0 | 0 | 0.5 |
| EMALEX GWIS-330EX | PEG-30 glyceryl-2 triisostearate | NIHON EMULSION Co., Ltd. | 10 | 10 | 10 |
| AJK-IS3613 | isostearic acid, dibutyl lauroyl glutamide, dibutyl ethyl hexanoyl glutamide | KOKYU ALCOHOL KOGYO Co., Ltd. | 10 | 10 | 10 |
| HICALL®K230 | Mineral oil | KANEDA Co., Ltd. | 52 | 47 | 27 |
| IPM-R | isopropyl myristate | KOKYU ALCOHOL KOGYO Co., Ltd. | 28 | 28 | 43 |
| Solidity | | | 0.13 | 0.11 | 0.11 |

### <Measuring Methods>

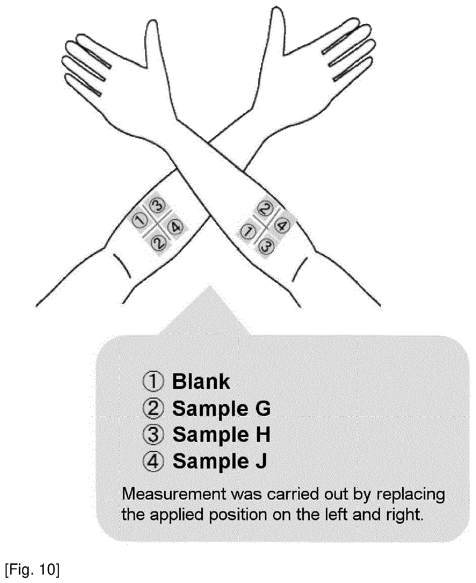

Measurement was carried out by a method that is similar to (1) except that Fig. 7 is replaced by Fig. 9.

### <Assessment Results>

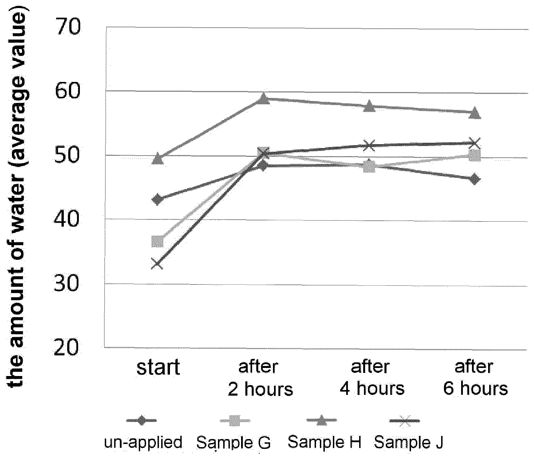

Results are shown in Fig. 10.

Samples were compared with un-applied control after equal time course, and Sample H (5% glycerin blended) after 2, 4 and 6 hours and Sample J (0.5% maltitol blended) after 6 hours showed results with significant difference in moisturizing effect.

### [Working Examples]

### Working Example 1. Stick form cleansing (leave-off type)

**[Table 12]**

| | | wt% |
|---|---|---|
| 1. | Isodecyl neopentanoate | 20.0 |
| | (Trade name: NEOLIGHT 100P, from KOKYU ALCOHOL KOGYO Co., Ltd.) | |
| 2. | Ethyl isostearate | 7.5 |
| | (Trade name: EIS-V, from KOKYU ALCOHOL KOGYO Co., Ltd.) | |
| 3. | Pentaerythrityl tetraisostearate | 5.0 |
| | (Trade name: KAK PTI, from KOKYU ALCOHOL KOGYO Co., Ltd.) | |
| 4. | Trimethylolpropane triisostearate | 5.0 |
| | (Trade name: KAK TTI, from KOKYU ALCOHOL KOGYO Co., Ltd.) | |
| 5. | Diglyceryl triisostearate | 27.0 |
| | (Trade name: RISOCAST PGIS, from KOKYU ALCOHOL KOGYO Co., Ltd.) | |
| 6. | PEG-20 glyceryl triisostearate | 25.0 |
| | (Trade name: EMALEX GWIS320, from NIHON EMULSION Co., Ltd.) | |
| 7. | 1,3-buthylene glycol | 7.5 |
| | (Trade name: HAISUGARCANE BG, from KOKYU ALCOHOL KOGYO Co., Ltd.) | |
| 8. | Deionized water | 3.0 |
| 9. | Perfumes | q.s. |

Preparation method: 1-6 and 9 are mixed homogeneously at 80 °C (oil phase). On the other hand, 7 and 8 are homogeneously dissolved at 60 °C (aqueous phase). To the oil phase at 80 °C, while being stirred, gradually added the aqueous phase at 60 °C to give a homogeneous mixture. This mixture was filled into a container and left to cool to room temperature to give a stick form cleansing of interest having 91 % transmittance.

### Working Example 2. Stick form cleansing (leave-off type)

**[Table 13]**

| | | wt% |
|---|---|---|
| 1. | Deionized water | 6.0 |
| 2. | 1,3-buthylene glycol | 5.0 |
| | (Trade name: HAISUGARCANE BG, from KOKYU ALCOHOL KOGYO Co., Ltd.) | |
| 3. | Stearoyloxy octyldodecyl stearate | 3.0 |
| | (Trade name: RISOCAST ODSHS, from KOKYU ALCOHOL KOGYO Co., Ltd.) | |
| 4. | PEG-20hydrogenated castor oil | 10.0 |
| | (Trade name: EMALEX HC-20, from NIHON EMULSION Co., Ltd.) | |
| 5. | Dibutyl lauroyl glutamide (27 %)/ | 15.0 |
| | Dibutyl ethyl hexanoyl glutamide (9 %)/ | |
| | Isostearic acid (64 %) mixture | |
| | (Trade name: AJK-IS3613, from KOKYU ALCOHOL KOGYO Co., Ltd.) | |
| 6. | Isobutyl isostearate | 10.0 |
| | (Trade name: KAK IBIS, from KOKYU ALCOHOL KOGYO Co., Ltd.) | |
| 7. | Tri(capryate/caprate) glyceryl | 5.0 |
| | (Trade name: TCG-M, from KOKYU ALCOHOL KOGYO Co., Ltd.) | |
| 8. | Mineral oil | 44.5 |
| 9. | Ceramide NP | 0.5 |
| 10. | Safflower essence | 1.0 |
| | (Trade name: safflower extract BG-50, from KOEI KOGYO Co., Ltd.) | |
| 11. | Perfumes | q.s. |

Preparation method: 3-9 and 11 are mixed homogeneously at 80°C (oil phase). On the other hand, 1, 2 and 10 are homogeneously dissolved at 60°C (aqueous phase). To the oil phase at 80 °C, while being stirred, gradually added the aqueous phase at 60 °C to give a homogeneous mixture. This mixture was filled into a container and left to cool to room temperature to give a stick form cleansing of interest having 83% transmittance.

### Working Example 3. Stick form anti-aging serum (leave-on type)

**[Table 14]**

| | | wt% |
|---|---|---|
| 1. | Deionized water | 7.5 |
| 2. | 1,3-buthylene glycol | 5.0 |
| | (Trade name: HAISUGARCANE BG, from KOKYU ALCOHOL KOGYO Co., Ltd.) | |
| 3. | Dipropylene glycol | 1.0 |
| 4. | Glycerin | 2.0 |
| | (Trade name: triol VE, from KOKYU ALCOHOL KOGYO Co., Ltd.)) | |
| 5. | Hexyl laurate | 15.0 |
| | (Trade name: KAK HL, from KOKYU ALCOHOL KOGYO Co., Ltd.) | |
| 6. | Dibutyl lauroyl glutamide (12 %)/ | 12.0 |
| | Dibutyl ethyl hexanoyl glutamide (8 %)/ | |
| | Octyldodecanol (80 %) mixture | |
| | (Trade name: AJK-OD2046, from KOKYU ALCOHOL KOGYO Co., Ltd.) | |
| 7. | Hydrogenated polyisobutene | 20.0 |
| | (Trade name: PARLEAM®6, from NOF CORPORATION) | |
| 8. | Hydroxystearyl ethylhexyl | 10.0 |
| | (Trade name: RISOCAST IOHS, from KOKYU ALCOHOL KOGYO Co., Ltd.) | |
| 9. | PEG-30 glyceryl triisostearate | 10.0 |
| | (Trade name: EMALEX 330, from NIHON EMULSION Co., Ltd.) | |
| 10. | Retinol acetate | 0.05 |
| 11. | Vitamin-E acetate | 0.5 |
| 12. | Oil-soluble Panax ginseng root essence | 0.1 |
| 13. | Perfumes | q.s. |

Preparation method: 5-13 are mixed homogeneously at 80 °C (oil phase). On the other hand, 1-4 are homogeneously dissolved at 60°C (aqueous phase). To the oil phase at 80°C, while being stirred, gradually added the aqueous phase at 60°C to give a homogeneous mixture. This mixture was filled into a container and left to cool to room temperature to give a stick form anti-aging serum of interest having 81 % transmittance.

### Working Example 4. Stick form whitening serum (leave-on type)

**[Table 15]**

| | | wt% |
|---|---|---|
| 1. | Deionized water | 10.0 |
| 2. | Glycerin | 1.0 |
| | (Trade name: triol VE, from KOKYU ALCOHOL KOGYO Co., Ltd.)) | |
| 3. | 1,3-buthylene glycol | 3.0 |
| | (Trade name: HAISUGARCANE, from KOKYU ALCOHOL KOGYO Co., Ltd.) | |
| 4. | Polyglyceryl-2 diisostearate | 10.0 |
| | (Trade name: RISOREX PGIS22, from KOKYU ALCOHOL KOGYO Co., Ltd.) | |
| 5. | Steareth-11 | 10.0 |
| | (Trade name: EMALEX 611(HLB11), from NIHON EMULSION Co., Ltd.) | |
| 6. | Dibutyl lauroyl glutamide (12%)/ | 10.0 |
| | Dibutyl ethyl hexanoyl glutamide (8%)/ | |
| | Octyldodecanol (80%) mixture | |
| | (Trade name: AJK-OD2046, from KOKYU ALCOHOL KOGYO Co., Ltd.) | |
| 7. | Mineral oil | 32.5 |
| 8. | Ethylhexyl isononanoate | 18.95 |
| | (Trade name: ES108109, from KOKYU ALCOHOL KOGYO Co., Ltd.) | |
| 9. | Vitamin-E acetate | 0.5 |
| 10. | Perfumes | q.s. |
| 11. | Tranexamic acid | 2.0 |
| 12. | Dipotassium glycyrrhizinate | 0.05 |
| 13. | Sponge gourd extract | 1.0 |
| 14. | Resveratrol extract | 1.0 |

Preparation method: 4-10 are mixed homogeneously at 80°C (oil phase). On the other hand, 1-3 and 11-14 are homogeneously dissolved at 60°C (aqueous phase). To the oil phase at 80°C, while being stirred, gradually added the aqueous phase at 60°C to give a homogeneous mixture. This mixture was filled into a container and left to cool to room temperature to give a stick form whitening serum of interest having 88% transmittance.

### Working Example 5. Stick form cleansing (leave-off type)

**[Table 16]**

| | | wt% |
|---|---|---|
| 1. | Isodecyl neopentanoate | 29.0 |
| | (Trade name: NEOLIGHT 100P, from KOKYU ALCOHOL KOGYO Co., Ltd.) | |
| 2. | Polyglyceryl-2 triisostearate | 9.0 |
| | (Trade name: RISOREX PGIS23, from KOKYU ALCOHOL KOGYO Co., Ltd.) | |
| 3. | Tricyclodecanemethyl isononanoate | 10.0 |
| | (Trade name: KAK TCIN, from KOKYU ALCOHOL KOGYO Co., Ltd.) | |
| 4. | Dipentaerythrityl hexaisononanoate | 10.0 |
| | (Trade name: HAILUCENT DPIN6, from KOKYU ALCOHOL KOGYO Co., Ltd.) | |
| 5. | PEG-20 glyceryl triisostearate | 25.0 |
| | (Trade name: EMALEX GWIS-320(HLB8), from NIHON EMULSION Co., Ltd.) | |
| 6. | Dibutyl lauroyl glutamide (12 %)/ | 10.0 |
| | Dibutyl ethyl hexanoyl glutamide (8 %)/ | |
| | Octyldodecanol (80 %) mixture | |
| | (Trade name: AJK-OD2046, from KOKYU ALCOHOL KOGYO Co., Ltd.) | |
| 7. | 1,3-buthylene glycol | 5.0 |
| | (Trade name: HAISUGARCANE BG, from KOKYU ALCOHOL KOGYO Co., Ltd.) | |
| 8. | Deionized water | 5.0 |
| 9. | *Angelica acutiloba* extract | 0.5 |
| 10. | Thyme extract | 0.5 |
| 11. | Perfumes | q.s. |

Preparation method: The mixture of 1-6 and 11 is mixed homogeneously at 80°C (oil phase). On the other hand, the mixture of 7-10 is heated to 60°C (aqueous phase). To the oil phase at 80°C, while being stirred, gradually added the aqueous phase at 60 °C to give a homogeneous mixture. This mixture was filled into a container and left to cool to room temperature to give a stick form cleansing of interest having 82 % transmittance.

As above, the present invention has been explained in details based on the suitable embodiments, though the present invention is not limited thereto, and each constitution can be substituted with any one that is capable of performing similar function, or any constitution can be added.

### [Industrial Applicability]

By utilizing solid form cosmetics comprising a gelling agent comprising dibutyl lauroyl glutamide and/or dibutyl ethyl hexanoyl glutamide, oil agent, surfactant and water of the present invention, it will be possible to provide solid form cosmetics with high transmittance having cleansing or skincare effect.

## Claims

1. A solid form cosmetic comprising a gelling agent comprising dibutyl lauroyl glutamide and/or dibutyl ethyl hexanoyl glutamide, oil agent, surfactant and water.

2. The solid form cosmetic according to Claim 1, wherein the transmittance is 80 % or higher.

3. The solid form cosmetic according to Claim 1 or 2, wherein the oil agent is a hydrocarbon oil, an ester oil having no hydroxy group, and/or a silicone oil which is not nonpolar.

4. The solid form cosmetic according to any one of Claims 1 to 3, wherein HLB of the surfactant is between 8 and 11.

5. The solid form cosmetic according to any one of Claims 1 to 4, wherein the content of dibutyl lauroyl glutamide is between 0.5 and 10.0 wt%, and the content of dibutyl ethyl hexanoyl glutamide is between 0.5 and 10.0 wt%.

6. The solid form cosmetic according to any one of Claims 1 to 5, wherein the content of the surfactant is between 5.0 and 15.0 wt%.

7. The solid form cosmetic according to any one of Claims 1 to 6, wherein the content of water is between 0.01 and 10.0 wt%.

8. The solid form cosmetic according to any one of Claims 1 to 7, further comprising isostearic acid or octyldodecanol.

9. The solid form cosmetic according to any one of Claims 1 to 8, wherein the blending ratio of dibutyl lauroyl glutamide to dibutyl ethyl hexanoyl glutamide is between 75:25 and 25:75.

10. The solid form cosmetic according to any one of Claims 1 to 9, wherein DSC solidifying temperature of the oil agent is 50 °C or higher in a mixture of 90.0 wt% of oil agent with 10.0 wt% of a mixture of 27 wt% of dibutyl lauroyl glutamide, 9 wt% of dibutyl ethyl hexanoyl glutamide and 64 wt% of isostearic acid.

11. The solid form cosmetic according to any one of Claims 1 to 10, further comprising an ester having a hydroxy group in 10.0 wt% or less.

12. The solid form cosmetic according to any one of Claims 1 to 11, wherein the solid form cosmetic is a leave-off type cosmetic.

13. The solid form cosmetic according to any one of Claims 1 to 12, wherein the solid form cosmetic is a leave-on type cosmetic.
